# EUROPÄISCHE PATENTSCHRIFT

(11) **EP 1 298 302 B1**
(45) Veröffentlichungstag und Bekanntmachung des Hinweises auf die Patenterteilung: **25.02.2004**
(21) Anmeldenummer: 02102322.1
(22) Anmeldetag: 06.09.2002
(51) Int. Cl.: F02D 41/02, F02P 5/15, G01N 33/00, F01N 11/00

(54) **Abgasreinigungsverfahren für Magerbrennkraftmaschinen**
Exhaust gas purifying method for lean burn engines
Procédé pour la purification de gaz d'échappement pour moteur à combustion de mélange pauvre

(30) Priorität: 28.09.2001 DE 10147983
(43) Veröffentlichungstag der Anmeldung: 02.04.2003
(73) Patentinhaber: SIEMENS AKTIENGESELLSCHAFT, 80333 München (DE)
(72) Erfinder: Rösel, Gerd, 93055, Regensburg (DE); Schwarz, Roland, 93173, Wenzenbach (DE); Zhang, Hong, 93105, Tegernheim (DE)

(56) Entgegenhaltungen:
- EP-A- 0 997 626
- DE-A- 19 823 921
- DE-A- 19 945 374
- US-A- 5 857 163

## Beschreibung

Die Erfindung bezieht sich auf ein Abgasreinigungsverfahren für eine magerbetreibbare Brennkraftmaschine, bei dem in einer Magerbetriebsphase der Brennkraftmaschine emittierte NOx-Verbindungen in einem im Abgastrakt der Brennkraftmaschine befindlichen NOx-Speicherkatalysator gespeichert werden, wobei eine Beladung des NOx-Speicherkatalysator mit NOx-Verbindungen stattfindet, die Brennkraftmaschine zeitweise in Regenerationsbetriebsphasen betrieben wird, in denen der NOx-Speicherkatalysator die gespeicherten NOx-Verbindungen katalytisch umsetzt und dadurch entladen wird, wobei eine Zustandsgröße des NOx-Speicherkatalysators mittels eines Modells ermittelt wird und die Regenerationsbetriebsphasen zustandsgrößenabhängig eingeleitet werden.

Um den Kraftstoffverbrauch von Kraftfahrzeugen weiter zu reduzieren, kommen immer häufiger Brennkraftmaschinen zum Einsatz, die mit magerem Kraftstoff/Luft-Gemisch betrieben werden können, da der Wirkungsgrad einer Brennkraftmaschine im Magerbetrieb besonders hoch ist. Zur Erfüllung geforderter Abgasgrenzwerte ist jedoch im Magerbetrieb einer Brennkraftmaschine regelmäßig eine spezifische Abgasnachbehandlung erforderlich, da ansonsten Grenzwerte hinsichtlich zulässiger NOx-Emissionen überschritten würden.

Deshalb werden NOx-Speicherkatalysatoren verwendet, die aufgrund einer besonderen Beschichtung in der Lage sind, NOx-Verbindungen aus dem Abgas zu absorbieren, die bei magerer Verbrennung entstehen. Zur Entleerung eines solchen NOx-Speicherkatalysators ist eine Regneration erforderlich, in der gespeicherte NOx-Verbindungen im Nox-Speicherkatalysator in unschädliche Verbindungen umgewandelt werden, wenn ein Reduktionsmittel zugegeben wird. Als Reduktionsmittel können Kohlenmonoxyd, Wasserstoff und Kohlenwasserstoff verwendet werden.

Es hat sich als zweckmäßig herausgestellt, diese durch kurzzeitigen Betrieb der Brennkraftmaschine mit einem fetten Kraftstoff/Luft-Gemisch zu erzeugen, wodurch der NOx-Speicherkatalysator das notwendige Reduktionsmittel als Bestandteil des ihm ohnehin zugeführten Abgases erhält und gespeicherte NOx-Verbindungen abbaut, so dass er wieder zur erneuten Einspeicherung von NOx-Verbindungen in der Lage ist. Dieser kurzzeitige Betrieb der Brennkraftmaschine stellt die erwähnte Regenerationsbetriebsphase dar.

Durch solche Regenerationsbetriebsphasen sinkt der Wirkungsgrad, d.h. der Kraftstoffverbrauch steigt. Darüber hinaus tritt während der Regenerationsbetriebsphase ein zwar oftmals nur geringer, jedoch prinzipiell unvermeidlicher Schlupf an Reduktionsmittel durch den NOx-Speicherkatalysator auf, wodurch in der Regenerationsbetriebsphase das Abgasverhalten der Brennkraftmaschine verschlechtert ist. Aus diesen Gründen möchte man Regenerationsbetriebsphasen nur dann einleiten, wenn sie wirklich zur Entleerung des NOx-Speicherkatalysators erforderlich sind.

Es ist deshalb der Stand der Technik bekannt, die Einleitung der Regenerationsbetriebsphasen bedarfsabhängig zu gestalten. Ein wesentliches Kriterium, wann eine Regenerationsbetriebsphase eingeleitet werden sollte, ist die gegenwärtige Speicherfähigkeit des NOx-Speicherkatalysators, die wiederum von der aktuell vorliegenden Beladung des NOx-Speicherkatalysators abhängt. Mit zunehmender Dauer der Magerbetriebsphase und dabei erfolgender Einspeicherung von NOx-Verbindungen nimmt der Speicherwirkungsgrad kontinuierlich ab, so dass unter Berücksichtigung der Abgasgrenzwerte eine Regenerationsbetriebsphase erforderlich wird.

Zur Einleitung einer Regenerationsbetriebsphase ist es im Stand der Technik bekannt, die NOx-Konzentration zu messen, so z.B. aus der DE 198 44 082 C1 der Anmelderin. Weiter kann der Sauerstoffgehalt im Abgasstrom stromab des NOx-Speicherkatalysators gemessen und damit eine am aktuellen Zustand des NOx-Speicherkatalysators orientierte Einleitung der Regenerationsbetriebsphase bewirkt werden. Gleichzeitig wird mit Hilfe der Messung des Sauerstoffgehaltes die Dauer einer Regenerationsbetriebsphase gesteuert. Optional wird in dieser Druckschrift vorgeschlagen, auch einen NOx-Messaufnehmer stromauf des NOx-Speicherkatalysators anzuordnen, um anhand tatsächlicher NOx-Konzentrationsmessungen den Betrieb der Brennkraftmaschine, also die Einleitung von Regenerationsbetriebsphasen zu bewirken.

Die Einhaltung aktueller oder zukünftiger Abgasgrenzwerte erfordert aber zunehmend eine Redundanz bzw. Selbstüberwachung emissionsrelevanter Bauteile im Rahmen der sogenannten On-Board-Diagnose. Dies macht bei einem Konzept gemäß DE 198 44 082 C1, bei dem die Einleitung von Regenerationsbetriebsphasen messsignalabhängig gestaltet wird, einen zusätzlichen Sensor bzw. besondere Sensorenüberwachung erforderlich.

Ein alternativer Ansatz zur Steuerung der Regenerationsbetriebsphasen nimmt Modellrechnungen vor, die eine Zustandsgeeignete Zustandsgröße, z. B. die aktuelle Beladung des NOx-Speicherkatalysators abschätzen.

So beschreibt beispielsweise die DE 195 17 168 A1, von der im Oberbegriff des Hauptanspruches ausgegangen wurde, die Abschätzung der in einem NOx-Speicherkatalysator eingespeicherten Menge an Nox-Verbindungen auf Basis der dem NOx-Speicherkatalysator im Rohabgas zugeführten NOx-Menge, welche wiederum ausgehend von den Betriebsparametern der Brennkraftmaschine in der Magerphase ermittelt wird. Dabei werden entsprechende Kennfelder verwendet, die unter anderem die Drehzahl, die Zündverhältnisse (Zündzeitpunkt) sowie den Lambda-Wert des Abgases berücksichtigen. Darüber hinaus spielt als wesentliches Kriterium die Temperatur des NOx-Speicherkatalysators eine Rolle, da diese sich stark auf die Adsorptionsverhältnisse im NOx-Speicherkatalysator und damit dessen Speicherwirkungsgrad auswirkt. Das in der DE 195 17 168 A1 beschriebene Modell sieht weiter vor, die vom NOx-Speicherkatalysator nicht gespeicherte, letztendlich von der Brennkraftmaschine emittierte NOx-Menge als Maß für die Einleitung einer Regenerationsbetriebsphase zu verwenden.

Ein ähnliches Modell ist aus der EP 0 597 106 A1 bekannt, bei dem ebenfalls die im NOx-Speicherkatalysator absorbierte Menge an NOx-Verbindungen in Abhängigkeit von Betriebsdaten der Brennkraftmaschine berechnet und bei Überschreiten einer bestimmten Grenzmenge an gespeicherten NOx-Verbindungen eine Regenerationsbetriebsphase eingeleitet wird.

Beide Modelle haben gemein, dass die Entscheidung, wann eine Regenerationsbetriebsphase eingeleitet werden soll, ausschließlich aufgrund bekannten Betriebsdaten der Brennkraftmaschine und des NOx-Speicherkatalysators erfolgt.

Die modellbasierten Verfahren benötigen zwar weniger Sensoraufwand, erreichen aber bei gleichem Kraftstoffverbrauch nicht die niedrigen Emissionswerte, wie das Verfahren gemäß DE 198 44 082 C1. Dieses ist dagegen für eine zuverlässige Funktion auf mehrfachen Sensoreinsatz angewiesen.

Der Erfindung liegt deshalb die Aufgabe zugrunde, ein Verfahren der eingangs genannten Art so weiterzubilden, dass eine verbesserte Abgasreinigung ohne mehrfachen Sensoreinsatz möglich wird.

Diese Aufgabe wird erfindungsgemäß bei einem gattungsgemäßen Verfahren dadurch gelöst, dass mittels des Modells eine Konzentration der NOx-Verbindungen stromab des NOx-Speicherkatalysators in Form eines NOx-Modellwertes ermittelt wird, stromab des NOx-Speicherkatalysators im Abgastrakt eine Konzentration der NOx-Verbindungen in Form eines NOx-Messwertes gemessen wird und die Differenz zwischen NOx-Messwert und NOx-Modellwert bestimmt und für eine Korrektur der im Modell verwendeten Zustandsgröße eingesetzt wird.

Die Erfindung verwendet also in Abkehr von den bisherigen Konzepten, die entweder eine modellbasierte oder eine messungsbasierte Einleitung der Regenerationsbetriebsphasen vorsahen, eine Kombination dieser beiden, bisher stark unterschiedlichen Konzepte vor. Zur Einleitung einer Regenerationsbetriebsphase wird in einem Modell der Beladungszustand des NOx-Speicherkatalysators ermittelt. Dazu können die aus dem Stand der Technik bekannten, vorerwähnten Modelle herangezogen werden. Zusätzlich erfolgt jedoch stromab des NOx-Speicherkatalysators eine NOx-Konzentrationsmessung, mit der das Modell korrigiert wird.

Dadurch wird die erforderliche Redundanz geschaffen, da bei Ausfall des Modells oder bei Ausfall der Messstelle stromab des NOx-Speicherkatalysators dennoch ein ausreichender Betrieb der Abgasreinigung sichergestellt ist.

Gegenüber einem bekannten messwertbasierten Verfahren besteht größere Unempfindlichkeit gegenüber Sensorausfällen oder fehlerbehafteten Sensorsignalen, da jederzeit die (abgeglichenen) NOx-Modellwerte zur Verfügung stehen. Dadurch kann auch bei kurzeitigem Sensorausfall eine Magerbetriebsphase sicher beendet und eine erforderliche Regenerationsbetriebsphase eingeleitet werden. Sicherheitsreserven bezüglich der Dauer einer Magerbetriebsphase, die ansonsten zur garantierten Einhaltung von Abgasgrenzwerten erforderlich wären, können verringert werden, wodurch der Kraftstoffverbrauch sinkt.

Die für die Erfindung geeigneten Modelle müssen nicht dieselbe Zustandsgröße zur Beurteilung, ob eine Regenerationsbetriebsphase eingeleitet werden muss, verwenden. Wesentlich ist nur, dass die Differenz in eine Zustandsgrößenkorrektur umgesetzt werden kann, z. B. durch ein Kennfeld.

So kann, wie eingangs geschildert, ein Modell den Beladungsgrad des NOx-Speicherkatalysators als Zustandsgröße auswerten oder die vom NOx-Speicherkatalysator nicht absorbierte und damit endgültig von der Abgasreinigungsanlage emittierte NOx-Menge als Zustandsgröße verwenden. In jedem Fall kann jedoch die verwendete Zustandsgröße, die zur Beurteilung, ob eine Regenerationsbetriebsphase eingeleitet werden soll, mit Hilfe der Differenz aus NOx-Modellwert und NOx-Messwert dahingehend korrigiert werden, dass die Regenerationsbetriebsphase zum optimalen Zeitpunkt eingeleitet werden kann. Etwaige modellbedingte Abweichungen werden damit durch den derart erfolgenden Modellabgleich korrigiert und spielen somit keine Rolle mehr.

Besonders zweckmäßig hat es sich erwiesen, den Beladungsgrad des NOx-Speicherkatalysators als Zustandsgröße zu verwenden, da dieser mit einem maximalen Beladungsgrad verknüpft zum einen eine zuverlässige Aussage über die Notwendigkeit einer Regenerationsbetriebsphase ermöglicht und zum anderen auch eine Diagnoseaussage über die Betriebsfähigkeit des NOx-Speicherkatalysators zulässt.

Modellbasierte Verfahren zur Bestimmung des Beladungszustandes eines NOx-Speicherkatalysators erlauben die Bereitstellung sehr aktueller Werte, wenn eine Berechnung mit entsprechend schnellen Aktualisierungszyklen erfolgt. Dann kann mit einem Modell ein sehr zeitaktueller Wert für die vom NOx-Speicherkatalysator und damit der Abgasreinigungsanlage abgegebene NOx-Verbindung erhalten werden. Messaufnehmer haben demgegenüber oft eine gewisse Totzeit, bis sie auf eine Änderung der NOx-Konzentration ansprechen. Dies ist besonders bei kostengünstigen Messaufnehmern der Fall, die regelmäßig nur ein stark tiefpassgefiltertes Messsignal abgeben. Für solche Kombinationen ist es zu bevorzugen, vor der Differenzbildung zwischen NOx-Messwert und NOx-Modellwert, den NOx-Modellwert tiefpass zu filtern, um die Aussagefähigkeit der Differenz zu steigern. Ansonsten würde ein zeitlich aktuellerer Wert in einer Differenz mit einem etwas zeitlich verzögerten Wert zusammengeführt.

Für den Fall, dass die Berechnungen im Modell mit einer geringeren Taktrate erfolgen, was unter dem Gesichtspunkt geringeren Rechenaufwands in einer Betriebssteuereinheit der Brennkraftmaschine erforderlich sein kann, kann es, insbesondere bei der Verwendung hochpräziser und schneller Messaufnehmer im Abgastrakt zu umgekehrten Verhältnissen kommen, d. h. der NOx-Modellwert kann zeitlich gegenüber dem NOx-Messwert verzögert sein. In einem solchen Fall ist eine Tiefpassfilterung des NOx-Messwertes zu bevorzugen.

Die erfindungsgemäß bestimmte Differenz kann auf verschiedene Weisen bei der modellabhängigen Steuerung der Regenerationsbetriebsphasen zur Korrektur des Modells berücksichtigt werden. Im wesentlichen hängt dies vom verwendeten Modell ab. Bei analytisch arbeitenden Modellen, die eine Rückrechnung von NOx-Konzentration auf den Beladungsgrad erlauben bzw. bei Modellen, die den NOx-Modellwert als Zustandsgröße verwenden, kann die Differenz direkt zur Korrektur des Modells eingesetzt werden, indem ein entsprechender Korrekturfaktor für die Zustandsgröße bei der Ermittlung des NOx-Modellwertes eingeführt wird, der von der berechneten Differenz abhängt.

Bei Modellen, die als wesentliche Bestandteile auf Kennfelder zugreifen, ist es zweckmäßig, die Differenz mittels eines Kennfeldes zur Korrektur der im Modell verwendeten Zustandsgröße einzusetzen. Diese kennfeldbasierte Berücksichtigung der Differenz kann auch bei anderen Modellen, die nicht wesentlich auf Kennfelder zur Einleitung von Regenerationsbetriebsphasen bauen, zur Korrektur zweckmäßig sein, da damit mitunter der Rechenaufwand gemindert wird.

Die Verwendung des errechneten Wertes der Differenz zwischen NOx-Modellwert und NOx-Messwert zur Durchführung der Korrektur des Modells kann in einem besonders einfachen Fall mittels eines Umrechnungsfaktors erfolgen, der mit dem errechneten Wert der Differenz multiplikativ verknüpft wird. Der Umrechnungsfaktor bildet somit eine proportionale Beziehung zwischen Differenz und zu korrigierender Zustandsgröße ab. Dieser lineare Absatz führt mit relativ geringem Aufwand zu einer verblüffend guten Korrektur.

In besserer Näherung kann diese Proportionalität zu einem beliebigen funktionalen Zusammenhang erweitert werden, indem der Umrechnungsfaktor einem Kennfeld entnommen wird. Da die Differenz zwischen NOx-Modellwert und NOx-Messwert im wesentlichen eine Aussage über den vom Modell bei der Beurteilung des Zustandes des NOx-Speicherkatalysators gemachten Fehler beinhaltet, ist es vorteilhaft, dieses Kennfeld abhängig von Größen zu wählen, die sich stark auf die Zustandsgröße auswirken. Dies sind in erster Linie die Temperatur des NOx-Speicherkatalysators sowie die die Konzentration an NOx-Verbindungen, die dem NOx-Speicherkatalysator mit dem Rohabgas zugeführt wird.

Es ist deshalb zu bevorzugen, das Kennfeld, dem der Umrechnungsfaktor entnommen wird, abhängig von NOx-Speicherkatalysatortemperatur und der Konzentration von NOx-Verbindungen im Rohabgas zu gestalten.

Die dabei in das Kennfeld eingehenden Größen können ihrerseits wieder aus einem Modell stammen, oder auch aus Messwerten gewonnen sein. Dies hängt von der Detailausgestaltung des verwendeten Modells ab. So sind Temperaturmodelle bekannt, um die wirkliche Temperatur des Katalysators, die sich auf seinen Wirkungsgrad auswirkt, zu bestimmen. Hierzu kann beispielsweise auf die DE 198 36 955 A1 der Anmelderin verwiesen werden.

Ähnliches gilt für die NOx-Konzentration im Rohabgas; auch hierzu sind Modelle bekannt, beispielsweise ein kennfeldbasierter Ansatz, wie er in der WO 98/55742 der Anmelderin beschrieben ist.

Die Verknüpfung der aus Umrechnungsfaktor und Differenz errechneten Korrekturgröße zur Zustandsgröße des Modells kann prinzipiell multiplikativ wie additiv erfolgen. Eine additive Verknüpfung hat jedoch den Vorteil, dass der Korrekturfaktor eine absolute Abweichung der Zustandsgröße wiedergibt und damit einen kontinuierlichen Modellversatz anzeigt, der sehr viel häufiger auftritt, als ein prozentualer Fehler. Darüber hinaus bietet sich bei additiver Verknüpfung die Möglichkeit, in einer Weiterbildung der Erfindung einen Offsetwert in Form eines Restwertes der Zustandsgröße bei vollständig regeneriertem NOx-Speicherkatalysator festzustellen.

Verwendet man als Zustandsgröße den Beladungsgrad, stellt der Modellversatz den Restbeladungsgrad dar, die sich beispielsweise anströmungsbedingt im NOx-Speicherkatalysator einstellt und auch dann vorliegt, wenn das Modell anzeigt, der NOx-Speicherkatalysator sei vollständig von NOx-Verbindungen entleert.

In einer bevorzugten Weiterbildung der Erfindung ist deshalb vorgesehen, dass direkt nach dem Ende einer Regnerationsphase eine Korrektur des im Modell verwendeten Beladungsgrades durchgeführt wird, wobei aus der Differenz zugleich ein Restbeladungsgrad des NOx-Speicherkatalysators ermittelt und dieser weiter im Modell berücksichtigt wird.

Die Erfindung wird nachfolgend unter Bezugnahme auf die Zeichnung beispielshalber noch näher erläutert. In der Zeichnung zeigt:
- Fig. 1: eine schematische Darstellung einer Brennkraftmaschine mit einem NOx-Speicherkatalysator,
- Fig. 2: ein schematisches Ablaufdiagramm zur Durchführung eines Verfahrens zur Abgasreinigung und
- Fig. 3: ein Ablaufdiagramm eines Abschnittes eines Verfahrens zur Abgasreinigung.

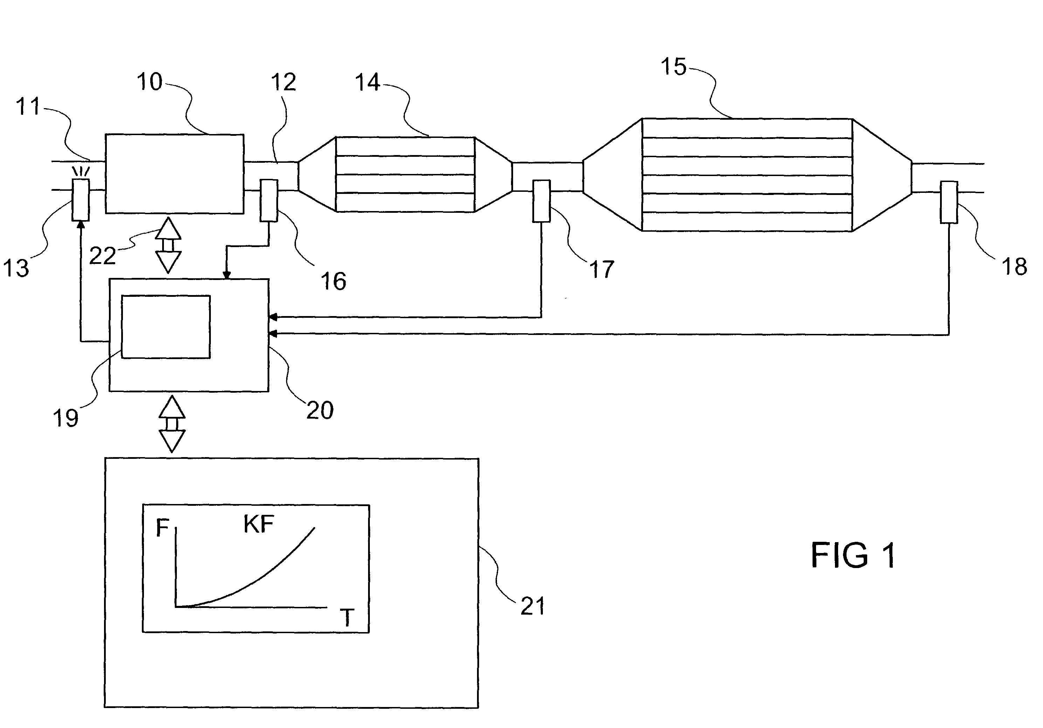

In Fig. 1 ist in Form eines Blockschaltbildes eine Brennkraftmaschine mit einer Abgasnachbehandlungsanlage gezeigt, bei der ein noch zu erläuterndes Verfahren zur Abgasreinigung eingesetzt wird. Dabei sind in der Fig. 1 nur diejenigen Bestandteile einer Brennkraftmaschine bzw. der Abgasnachbehandlungsanlage gezeigt, die für das Verständnis des Verfahrens erforderlich sind.

Eine Brennkraftmaschine 10 weist einen Ansaugtrakt 11 und einen Abgastrakt 12 auf. Im Ansaugtrakt 11 ist eine Kraftstoffzumesseinrichtung vorhanden, für die stellvertretend schematisch ein Einspritzventil 13 eingezeichnet ist. Das Einspritzventil 13 spritzt in den Ansaugtrakt 11 Kraftstoff ein. Alternativ zur Einspritzung in den Ansaugtrakt kann der Kraftstoff auch direkt in die Zylinder der Brennkraftmaschine in Form einer Direkteinspritzung eingebracht werden. Im Abgastrakt 12, in den die Brennkraftmaschine 10 ihr Abgas abgibt, ist eine Abgasnachbehandlungsanlage vorgesehen. Sie weist einen möglichst nahe der Brennkraftmaschine angeordneten Drei-Wege-Katalysator 14 auf, dem in Strömungsrichtung des Abgases ein NOx-Speicherkatalysator 15 nachgeschaltet ist.

Aufgrund seiner Lage vor dem NOx-Speicherkatalysator 15 wird der Drei-Wege-Katalysator auch als Vorkatalysator bezeichnet. Die Auswahl und Auslegung dieses Vorkatalysators erfolgt hinsichtlich schnellem Ansprechverhalten und Sauerstoffspeicherkapazität. Damit während des noch zu erläuternden Fettbetriebes in einer Regenerationsbetriebsphase bereitgestelltes Regenerationsmittel möglichst unvermindert dem NOx-Speicherkatalysator 15 zugeführt wird, sollte der Vorkatalysator eine möglichst geringe Sauerstoffspeicherkapazität aufweisen. Darüber hinaus sollte er eine möglichst weitgehende Oxidation von Kohlenwasserstoffen und Kohlenmonoxyd durchführen, da sich dies in Magerbetriebsphasen der Brennkraftmaschine 10 günstig auf die Arbeit des NOx-Speicherkatalysators auswirkt.

Der NOx-Speicherkatalysator 15 speichert in Magerbetriebsphasen der Brennkraftmaschine abgegebene NOx-Verbindungen. Er weist weiter eine Beschichtung auf, die bei stöchiometrischem Betrieb der Brennkraftmaschine 10 dem NOx-Speicherkatalysator 15 auch katalytische Drei-Wege-Funktion verleiht.

Zum Betrieb der Abgasnachbehandlungsanlage sind Sensoren vorgesehen, die einen Sauerstoffmessaufnehmer 16 stromaufwärts des Vorkatalysators, einen Temperatursensor 17 nahe dem NOx-Speicherkatalysator 15 und einen weiteren Sauerstoffmessaufnehmer 18 stromabwärts des NOx-Speicherkatalysators 15 umfassen.

Alle Sensoren sowie die Kraftstoffzumesseinrichtung sind über nicht näher bezeichnete Leitungen mit einem Steuergerät 20 verbunden, das einen Lambdaregler 19 enthält, der später noch näher erläutert wird.

Als Sauerstoffmessaufnehmer 16 wird vorzugsweise eine Breitband-Lambdasonde eingesetzt, die in Abhängigkeit vom Sauerstoffgehalt im Abgas ein stetiges, z.B. ein lineares Ausgangssignal abgibt. Aus dem Signal dieser Breitband-Lambdasonde 16 bestimmt das Steuergerät 20 sowohl während des Magerbetriebes als auch während der Regenerationsbetriebsphase die Luftzahl, mit der die Brennkraftmaschine 10 bestimmt wurde. Dabei wird der Lambdaregler 19 aktiv, der den Lambdawert im Betrieb der Brennkraftmaschine 10 entsprechend Sollvorgaben regelt. Die Regelung im optimalen Lambda-Bereich während stöchiometrischem Betrieb der Brennkraftmaschine mittels des Sauerstoffmessaufnehmers 18, der vorzugsweise als binäre Lambdasonde (2-Punkt-Lambda-Sonde) ausgebildet, ist eine bekannte Führungsregelung auf einen festen Lambdawert nahe 1. Dazu kann auch eine zwischen Vorkatalysator und NOx-Speicherkatalysator angeordnete Lambda-Sonde verwendet werden.

Im Sauerstoffmessaufnehmer 18 ist gleichzeitig ein NOx-Messaufnehmer integriert. Solche Messaufnehmer sind beispielsweise aus der Veröffentlichung N. Kato et al., "Performance of thick film NOx-sensor on diesel and gasoline engines", Society of Automotive Engineers, Publ. Nr. 970858, bekannt. Ein solcher Messaufnehmer gibt sowohl ein einen Lambda-Wert anzeigendes Signal auch ein entsprechendes Signal für die NOx-Konzentration im Abgas ab. Nachfolgend wird deshalb von einem NOx-Messaufnehmer 18 gesprochen.

Die Temperatur des NOx-Speicherkatalysators, die im nachfolgenden Verfahren ausgewertet ist, wird aus dem Signal des Temperatursensors 17 mittels eines Temperaturmodells errechnet, da der Temperatursensor 17 nur die Temperatur des in den NOx-Speicherkatalysator einströmenden Abgases anzeigt, welches sich von der Temperatur im NOx-Speicherkatalysator unterscheidet. Alternativ könnte die Temperatur des NOx-Speicherkatalysators 15 auch direkt gemessen werden, indem beispielsweise ein Temperatursensor unmittelbar am oder im Gehäuse des NOx-Speicherkatalysators 15 angeordnet wird.

Das Steuergerät 20 ist weiter über eine nicht näher bezeichnete Datenleitung mit einem Kennfeldspeicher 21 verbunden, in dem entsprechende Kennfelder, auf die noch zu sprechen kommen sein wird, abgelegt sind.

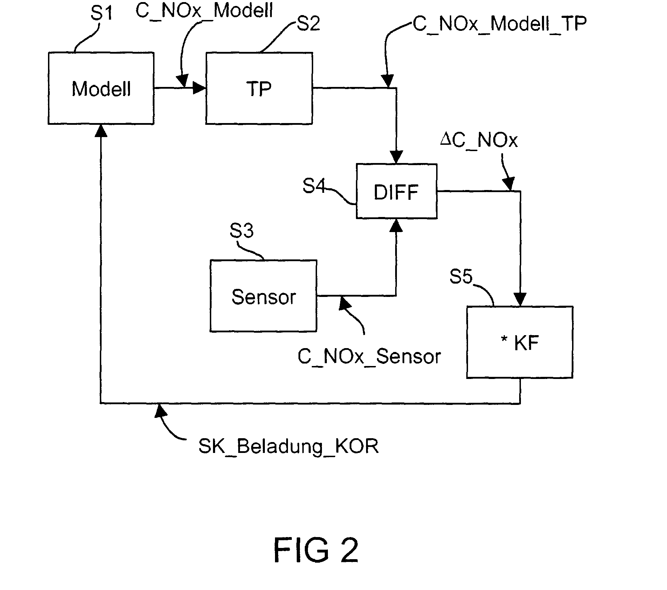

Zur Einleitung der Regenerationsbetriebsphasen der Brennkraftmaschine 10, in denen diese mit fettem Kraftstoff/Luft-Gemisch betrieben wird, um das für die Regeneration des NOx-Speicherkatalysators 15 erforderliche Regenerationsmittel im Rohabgas der Brennkraftmaschine 10 bereitzustellen, wird ein Modell verwendet, das an sich bekannt ist. Hierzu wird vollumfänglich auf die DE 196 07 151 C1 der Anmelderin verwiesen. Wesentlich für das Verständnis des nachfolgenden Verfahrens ist hierbei, dass dieses Modell den Beladungsgrad des NOx-Speicherkatalysators als Zustandsgröße auswertet, ob eine Regenerationsbetriebsphase eingeleitet werden soll. Weiter liefert dieses Modell, das in Fig. 2 schematisch mit einem Block S1 bezeichnet ist, einen NOx-Modellwert C_NOx_Modell für die vom NOx-Speicherkatalysator 15 abgegebene NOx-Konzentration.

C-NOx-Modell wird in einem Schritt S2 einer Tiefpassfilterung mit einem Tiefpassfilter TP unterzogen, da aufgrund des hohen Rechentaktes des Steuergerätes 20 der NOx-Modellwert C_NOx_Modell sehr aktuell ist, aktueller als der vom NOx-Messaufnehmer 18, der in Fig. 2 als Block S3 dargestellt ist, gelieferte NOx-Messwert C_NOx_Sensor.

Der Tiefpassfilter 10 ist in seinen Filtereigenschaften auf die Ansprechgeschwindigkeit des NOx-Messaufnehmers 18 abgestimmt. Dabei wird auch die Gaslaufzeit zwischen Ausgang des NOx-Speicherkatalysators 15 und dem NOx-Messaufnehmer 18 berücksichtigt. Insbesondere kann der Tiefpassfilter TP dazu betriebsparameterabhängig ausgelegt werden.

Der vom Tiefpassfilter TP abgegebene tiefpassgefilterte Wert C_NOx_Modell_TP wird in einem Schritt S4 mit dem NOx-Messwert C_NOx_Sensor in Form einer Differenzbildung verknüpft. Dadurch wird die Konzentrationsdifferenz ΔC_NOx erhalten. ΔC_NOx stellt die Abweichung der modellbasierten Aussage über die vom NOx-Speicherkatalysator 15 abgegebene NOx-Konzentration vom NOx-Messwert dar.

Die Konzentrationsdifferenz ΔC_NOx wird dann in einem Schritt S5 mit einem Faktor F verknüpft, der dem Kennfeldspeicher 21 entnommen wurde. Nach Durchführung des Schrittes S5 steht ein Korrekturfaktor SK-Beladung-KOR zur Verfügung, der mit dem im Modell verwendeten Beladungsgrad des NOx-Speicherkatalysators 15 verknüpft werden kann, um eine Korrektur zu bewirken.

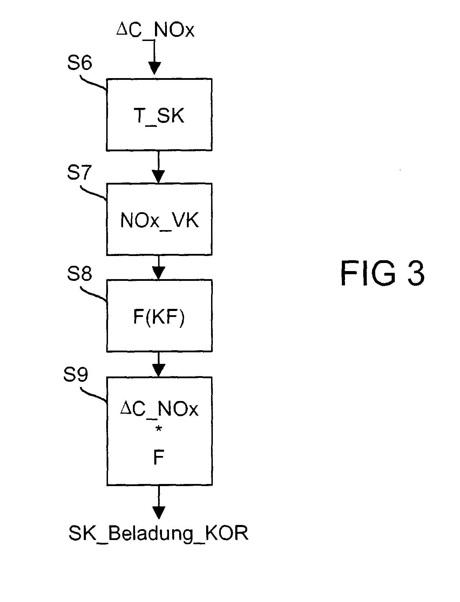

Die Gewinnung dieses Korrekturfaktors SK-Beladung-KOR ist in Fig. 3 detaillierter dargestellt.

Fig. 3 zeigt Teilschritte S6 bis S9, die in Fig. 2 im Schritt S5 zusammengefasst sind. Zuerst wird in einem Schritt S6 die Temperatur T-SK des NOx-Speicherkatalysators 15 ermittelt. Dazu wird unter Rückgriff auf den Temperatursensor 17 mittels des eingangs erwähnten Temperaturmodells, wie es in der hier ebenfalls vollumfänglich einbezogenen DE 198 36 955 A1 der Anmelderin beschrieben ist, die Temperatur T-SK des NOx-Speicherkatalysators festgestellt. Weiter wird in einem Schritt S7 die dem NOx-Speicherkatalysator zugeführte Vorkat-NOx-Konzentration NOx_VK aus aktuellen Werten von Betriebsparametern der Brennkraftmaschine berechnet und ggf. gefiltert. Hierzu wird wiederum auf den Kennfeldspeicher 21 zugegriffen.

Nun wird in einem Schritt S8 aus einem Kennfeld KF ein Wert für den Faktor F entnommen, der von der Temperatur T_SK und der Vorkat-NOx-Konzentration NOx_VK abhängt. Der derart erhaltene Faktor F wird dann multiplikativ mit der Konzentrationsdifferenz ΔC_NOx verknüpft, wodurch der Korrekturfaktor SK_Beladung_KOR erhalten wird.

Der Korrekturfaktor SK_Beladung_KOR wird dann im Modell zur additiven Korrektur des dort verwendeten Beladungsgrades eingesetzt. Durch diesen Schleifenschluss erfolgt eine Regelung des Modells derart, dass im Endeffekt die Konzentrationsdifferenz ΔC_NOx verschwindet, so dass das Modell optimal korrigiert ist. Die Korrektur kann allerdings auch an Randbedingungen geknüpft werden, z. B. an ein die aktuelle Verfügbarkeit des NOx-Messaufnehmer anzeigendes Signal, Zeit- bzw. Häufigkeitsbedingungen, nach denen eine Korrektur nur in bestimmten Abständen zulässig ist, oder an zeitliche Zusammenhänge bezüglich der letzten Regenerationsphase.

Weiter eröffnet dieses Konzept eine Plausibilisierung der Regelung derart, dass bei zu großen Konzentrationsdifferenzen ΔC_NOx eine weitere Betriebssteuerung wahlweise nur unter Rückgriff auf den NOx-Messwert oder den NOx-Modellwert erfolgt. Diese Regelschleife kann zusätzlich um einen Integralanteil erweitert werden, so dass die Abweichung zwischen NOx-Modellwert und NOx-Messwert mittels eines PI-Reglers zu Null geregelt wird. Für den I-Anteil gilt dabei das für den den P-Anteil darstellenden Faktor F gesagte sinngemäß.

In einer weiteren Anwendung des eingangs geschilderten Verfahrens kann aus dem Korrekturfaktor SK_Beladung_KOR eine Restbeladung des NOx-Speicherkatalysators 15 nach Durchführung einer Regenerationsbetriebsphase ermittelt werden.

Dazu wird das in Fig. 2 dargestellte Verfahren direkt nach Schluss einer Regenerationsbetriebsphase und zu Beginn einer Magerbetriebsphase ausgeführt. Der Schluss einer Regenerationsbetriebsphase kann entweder wiederum rein modellbasiert oder wie in der erwähnten DE 198 44 082 C1 beschrieben, gewählt werden. Der direkt nach Durchführen einer solchen Regenerationsbetriebsphase ermittelte Restbeladungsgrad des NOx-Speicherkatalysators 15 gibt Aussage über eine NOx-Menge, die aus dem NOx-Speicherkatalysator in einer normalen Regenerationsbetriebsphase nicht mehr desorbiert werden kann.

Dieser Restbeladungsgrad kann als nachfolgend bei der Ausführung des Modells fest berücksichtigte Größe Einsatz finden. Alternativ kann bei einem zu hoch ansteigenden Restbeladungsgrad bzw. bei einem Korrekturfaktor SK-Beladung-KOR, der einen bestimmten Schwellenwert überschreitet, eine besondere Regeneration eingeleitet werden, z. B. eine verlängerte Regenerationsbetriebsphase.

## Patentansprüche

1. Abgasreinigungsverfahren für eine magerbetreibbare Brennkraftmaschine, bei dem
- in einer Magerbetriebsphase der Brennkraftmaschine emittierte NOx-Verbindungen in einem im Abgastrakt der Brennkraftmaschine befindlichen NOx-Speicherkatalysator gespeichert werden, wobei eine Beladung des NOx-Speicherkatalysator mit NOx-Verbindungen stattfindet,
- die Brennkraftmaschine zeitweise in Regenerationsbetriebsphasen betrieben wird, in denen der NOx-Speicherkatalysator die gespeicherten NOx-Verbindungen katalytisch umsetzt und dadurch entladen wird,
- wobei eine Zustandsgröße des NOx-Speicherkatalysators mittels eines Modells ermittelt wird und die Regenerationsbetriebsphasen zustandsgrößenabhängig eingeleitet werden,
**dadurch gekennzeichnet, dass**
- mittels des Modells eine Konzentration der NOx-Verbindungen stromab des NOx-Speicherkatalysators in Form eines NOx-Modellwertes ermittelt wird,
- stromab des NOx-Speicherkatalysators im Abgastrakt eine Konzentration der NOx-Verbindungen in Form eines NOx-Messwertes gemessen wird und
- die Differenz zwischen NOx-Messwert und NOx-Modellwert bestimmt und für eine Korrektur der im Modell verwendeten Zustandsgröße eingesetzt wird.

2. Verfahren nach Anspruch 1, **dadurch gekennzeichnet, dass** der NOx-Messwert oder der NOx-Modellwert vor der Differenzbildung tiefpassgefiltert wird.

3. Verfahren nach einem der obigen Ansprüche, **dadurch gekennzeichnet, dass** der Beladungsgrad des NOx-Speicherkatalysators als Zustandsgröße verwendet wird.

4. Verfahren nach einem der obigen Ansprüche, **dadurch gekennzeichnet, dass** die Differenz mittels eines Kennfeldes zur Korrektur der im Modell verwendeten Zustandsgröße eingesetzt wird.

5. Verfahren nach einem der obigen Ansprüche, **dadurch gekennzeichnet, dass** die Differenz mit einem Umrechnungsfaktor multiplikativ zu einer Korrekturgröße für die im Modell verwendete Zustandsgröße verknüpft wird.

6. Verfahren nach den Ansprüchen 3, 4 und 5, **dadurch gekennzeichnet, dass** der Umrechnungsfaktor einem Kennfeld entnommen wird, das abhängt von NOx-Speicherkatalysatortemperatur und Konzentration von NOx-Verbindungen stromauf des NOx-Speicherkatalysators.

7. Verfahren nach Anspruch 5, **dadurch gekennzeichnet, dass** die Korrekturgröße additiv mit dem im Modell verwendeten Beladungsgrad verknüpft wird.

8. Verfahren nach einem der obigen Ansprüche in Verbindung mit Anspruch 7, **dadurch gekennzeichnet, dass** direkt nach dem Ende einer Regenerationsbetriebsphase eine Korrektur des im Modell verwendeten Beladungsgrades durchgeführt wird, wobei aus der Differenz zugleich ein Restbeladungsgrad des NOx-Speicherkatalysators ermittelt und dieser weiter im Modell berücksichtigt wird.

## Claims

1. Exhaust emission control method for a lean-burn internal combustion engine, whereby
- NOx compounds emitted in a lean-burn operating phase of the internal combustion engine are stored in a NOx storage catalytic converter located in the exhaust tract of the internal combustion engine, whereby the NOx storage catalytic converter becomes charged with NOx compounds,
- the internal combustion engine is intermittently operated in regeneration operating phases in which the NOx storage catalytic converter catalytically converts the stored NOx compounds and is thus discharged,
- whereby a state variable of the NOx storage catalytic converter is determined by means of a model and the regeneration operating phases are initiated in accordance with the state variable,
**characterised in that**,
- by means of the model, a concentration of NOx compounds is calculated downstream from the NOx storage catalytic converter in the form of a NOx model value,
- a concentration of NOx compounds is measured in the exhaust tract downstream from the NOx storage catalytic converter in the form of a NOx measured value, and
- the difference between NOx measured value and NOx model value is determined and used to correct the state variable used in the model.

2. Method according to Claim 1, **characterised in that** the NOx measured value or the NOx model value is low-pass filtered before the difference is calculated.

3. Method according to one of the above claims, **characterised in that** the charge level of the NOx storage catalytic converter is used as the state variable.

4. Method according to one of the above claims, **characterised in that** the difference is set by means of a characteristic map for correction of the state variable used in the model.

5. Method according to one of the above claims, **characterised in that** the difference is linked by multiplication to a conversion factor to produce a correction variable for the state variable used in the model.

6. Method according to Claims 3, 4 and 5, **characterised in that** the conversion factor is taken from a characteristic map that depends on the NOx storage catalytic converter temperature and concentration of NOx compounds upstream from the NOx storage catalytic converter.

7. Method according to Claim 5, **characterised in that** the correction variable is linked by addition to the charge level used in the model.

8. Method according to one of the above claims in combination with Claim 7, **characterised in that** a correction of the charge level used in the model is carried out immediately after the end of a regeneration operating phase, wherein a residual charge level of the NOx storage catalytic converter is calculated at the same time from the difference and is further taken into account in the model.

## Revendications

1. Procédé d'épuration des gaz d'échappement destiné à un moteur à combustion interne capable de fonctionner avec un mélange gazeux pauvre, lors duquel
- des composés de NOx émis par le moteur à combustion interne dans une phase où celui-ci fonctionne avec un mélange gazeux pauvre sont emmagasinés dans un catalyseur accumulateur de NOx situé dans le bloc pour gaz d'échappement du moteur à combustion interne, provoquant ainsi un, chargement du catalyseur accumulateur de NOx en composés de NOx,
- le moteur à combustion interne fonctionne momentanément dans des phases de régénération, pendant lesquelles le catalyseur accumulateur de NOx transforme de manière catalytique les composés de NOx emmagasinés et se trouve ainsi déchargé,
- une variable d'état du catalyseur accumulateur de NOx étant déterminée au moyen d'un modèle et les phases de régénération étant initialisées en fonction de ladite variable d'état,
**caractérisé en ce que**
- au moyen du modèle, une concentration de composés de NOx est déterminée en aval du catalyseur accumulateur de NOx sous la forme d'une valeur de modèle de NOx,
- en aval du catalyseur accumulateur de NOx situé dans le bloc pour gaz d'échappement est mesurée une concentration des composés de NOx sous la forme d'une valeur mesurée de NOx et
- la différence entre la valeur mesurée de NOx et la valeur de modèle de NOx est déterminée et utilisée pour corriger la variable d'état utilisée dans le modèle.

2. Procédé selon la revendication 1, **caractérisé en ce que** la valeur mesurée de NOx ou la valeur de modèle de NOx est soumise à un filtrage passe-bas avant que la formation de la différence soit effectuée.

3. Procédé selon l'une des revendications précédentes, **caractérisé en ce que** le degré de chargement du catalyseur accumulateur de NOx est utilisé comme variable d'état.

4. Procédé selon l'une des revendications précédentes, **caractérisé en ce que** la différence est utilisée au moyen d'un diagramme caractéristique pour corriger la variable d'état utilisée dans le modèle.

5. Procédé selon l'une des revendications précédentes, **caractérisé en ce que** la différence est liée de manière multiplicative à un facteur de conversion permettant d'obtenir une grandeur de correction pour la variable d'état utilisée dans le modèle.

6. Procédé selon les revendications 3, 4 et 5, **caractérisé en ce que** le facteur de conversion est prélevé dans un diagramme caractéristique qui dépend de la température du catalyseur accumulateur de NOx et de la concentration de composés de NOx en amont du catalyseur accumulateur de NOx.

7. Procédé selon la revendication 5, **caractérisé en ce que** la grandeur de correction est liée de manière additive au degré de chargement utilisé dans le modèle.

8. Procédé selon l'une des revendications précédentes en relation avec la revendication 7, **caractérisé en ce que**, immédiatement après la fin d'une phase de régénération, est réalisée une correction du degré de chargement utilisé dans le modèle, la différence servant en même temps à déterminer le degré de chargement résiduel du catalyseur accumulateur de NOx, qui sera ensuite pris en considération dans le modèle.
